# EUROPEAN PATENT SPECIFICATION

(11) **EP 3 760 249 B1**
(45) Date of publication and mention of the grant of the patent: **18.09.2024**
(21) Application number: 19777548.9
(22) Date of filing: 27.03.2019
(51) Int. Cl.: A61M 5/145

(54) **DRUG SOLUTION ADMINISTRATION DEVICE**
VORRICHTUNG ZUR VERABREICHUNG VON ARZNEIMITTELLÖSUNGEN
DISPOSITIF D'ADMINISTRATION DE SOLUTION DE MÉDICAMENT

(30) Priority: 30.03.2018 JP 2018067438
(43) Date of publication of application: 06.01.2021
(73) Proprietor: TERUMO Kabushiki Kaisha, Tokyo 151-0072 (JP)
(72) Inventor: KONDO, Akira, Ashigarakami-gun, Kanagawa 259-0151 (JP)
(74) Representative: TBK
(86) International application number: PCT/JP2019/013311
(87) International publication number: WO 2019/189436

(56) References cited:
- WO-A1-02/056947
- WO-A1-2010/110429
- WO-A1-2017/062931
- JP-A- 2011 217 817

## Description

### Technical Field

The present invention relates to a drug solution administration device.

### Background Art

Conventionally, a syringe pump-type drug solution administration device has been known that administers a drug solution filled in a drug solution container into a living body by a pressing action of a plunger. A drug solution administration device of this type includes a cylindrical drug solution container and a plunger mechanism for expelling the drug solution in the drug solution container (see, for example, document JP H09 - 294 807 A). The drug solution container and the plunger mechanism are housed in a housing and supported by the housing.

Document WO 02 / 056 947 A1 pertains to Syringe Interfaces and adapters for use with medical injectors. Here, a syringe having a flange at its end opposite a needle end is inserted into a housing. The flange serves to position the syringe by being accommodated in a receiving groove provided in a housing of the injector.
Document WO 2017 / 062 931 A1 is considered closest prior art and pertains to an add-on for self-injectors. Here, a syringe having a flange at its end opposite a needle end is put into a housing and secured by means of fasteners.

### Summary of Invention

### Technical Problem

In the abovementioned drug solution administration device, when the plunger advances in the drug solution container to deliver the drug solution, thrust is generated with the forward movement of the plunger, so that an excessive pressing force may be applied to the plunger and the drug solution container. As a result, the plunger and the drug solution container deform so as to bend with respect to the direction of forward movement of the plunger (the longitudinal direction of the plunger and the drug solution container), which may entail a problem that smooth delivery of the drug solution is inhibited.

The present invention has been accomplished in view of the above problems, and the object of the present invention is to provide a drug solution administration device capable of preventing deformation resulting in bending of a plunger and a drug solution container during delivery of a drug solution.
The object of the invention is achieved by a drug solution administration device according to claim 1. Advantageous embodiments are carried out according to the dependent claims.

### Solution to Problem

The drug solution administration device according to the present invention includes: a drug solution container that has a main body having a cylindrical shape and filled with a drug solution; a housing that holds the drug solution container; a plunger that expels the drug solution in the drug solution container; and a fixing member that fixes the drug solution container to the housing, wherein the housing includes a support part having a support surface that supports a bottom surface of the main body of the drug solution container, and the fixing member includes a guide part mounted along the main body of the drug solution container from a side opposite to the bottom surface of the main body of the drug solution container in a circumferential direction, and an engagement part engageable with the support part of the housing.

### Advantageous Effects of Invention

The drug solution administration device according to the present invention can prevent deformation resulting in bending of the plunger and the drug solution container during delivery of the drug solution.

### Brief Description of Drawings

Fig. 1 is a side view of a drug solution administration system according to an embodiment.
Fig. 2 is a diagram schematically showing an example of use of the drug solution administration system.
Fig. 3 is a schematic perspective view of a drug solution administration device.
Fig. 4 is a schematic perspective view of a chassis included in a housing and components attached to the chassis.
Fig. 5 is a schematic perspective view of a fixing member.
Fig. 6 is a perspective view showing a state before the fixing member is attached to the chassis.
Fig. 7 is a perspective view showing a way of attaching the fixing member to the chassis.
Fig. 8 is an enlarged view showing a way of attaching the fixing member to the chassis.
Fig. 9 is an enlarged view showing a state after the fixing member is attached to the chassis.
Fig. 10 is a perspective view showing holes formed in the chassis.

### Description of Embodiments

An embodiment of the present invention will now be described with reference to the accompanying drawings. It should be noted that the following description is not intended to limit the technical scopes and meanings of terms set forth in the claims of the present application. Further, the dimensional ratios in the drawings are exaggerated for convenience of description, and may differ from the actual ratios.

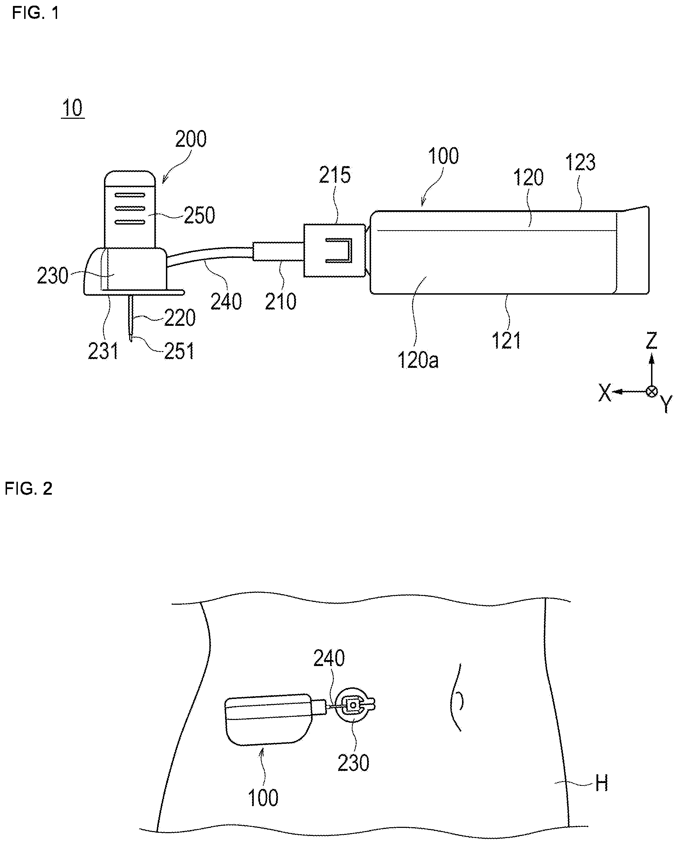

Figs. 1 to 6 are diagrams for describing a drug solution administration system 10, a drug solution administration device 100, and an administration instrument 200 according to the embodiment. An arrow X in each drawing indicates the "longitudinal direction (longitudinal direction of a drug solution container 110)" of the drug solution administration device 100, an arrow Y indicates the "width direction (depth direction)" of the drug solution administration device 100, and an arrow Z indicates the "height direction" of the drug solution administration device 100.

### (Drug solution administration system)

The drug solution administration system 10 is used to administer a drug solution into a living body. As shown in Fig. 1, the drug solution administration system 10 includes the drug solution administration device 100 and the administration instrument 200.

As shown in Fig. 2, the drug solution administration device 100 and the administration instrument 200 are of a patch type that is attached to the body surface (skin) H of a user when used. The body part of the user to which the drug solution administration device 100 and the administration instrument 200 are attached is not particularly limited, and examples thereof include the abdomen and the thigh.

The drug solution administration system 10 can continuously administer a drug solution (not shown) filled in the drug solution container 110 included in the drug solution administration device 100 into a living body for relatively a long period of time (for example, several minutes to several hours) by a pressing action of a later-described plunger 130. Note that the drug solution administration system 10 may administer the drug solution at intervals into the living body.

### (Drug solution administration device)

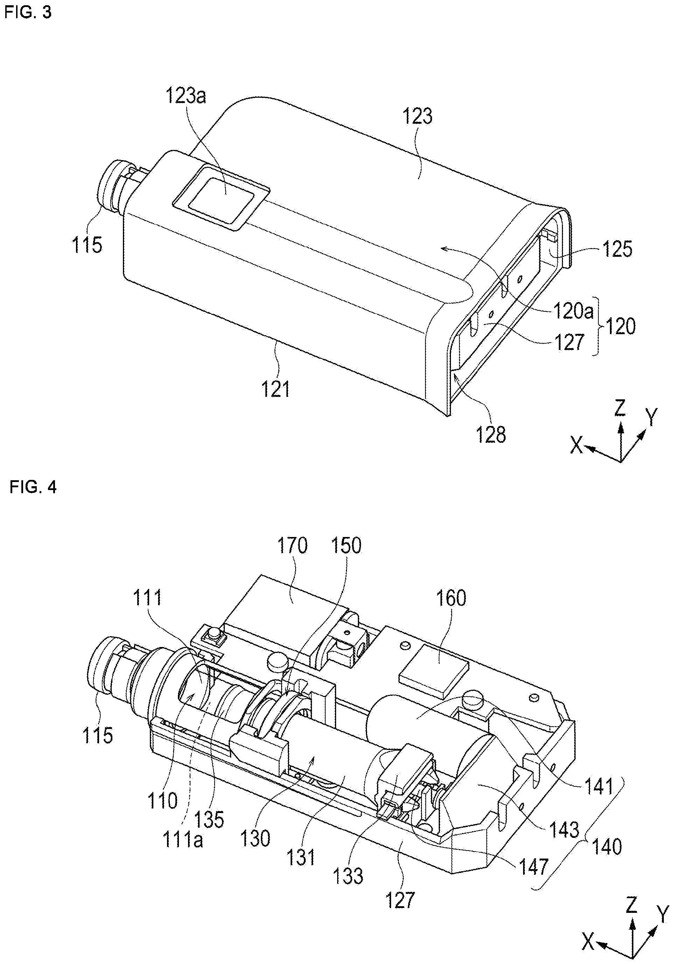

As shown in Figs. 3 and 4, the drug solution administration device 100 includes the drug solution container 110 having a cylindrical (barrel-shaped) main body 111 filled with a drug solution, a housing 120 for holding the drug solution container 110, a plunger 130 for expelling the drug solution in the drug solution container 110, and a fixing member 150 for fixing the drug solution container 110.

As shown in Figs. 3 and 4, the housing 120 has a box-shaped housing body 120a having a housing space 128 formed therein, and a chassis 127 (corresponding to a "support part") that is housed in the housing space 128 of the housing body 120a and can be fixed to the housing body 120a.

As shown in Fig. 3, a window 123a is formed in an upper surface 123 of the housing body 120a to allow the inside of the housing space 128 to be visible from the outside of the housing 120. The window 123a is formed by providing a transparent or semitransparent portion on a part of the housing body 120a.

A base end opening 125 for inserting the chassis 127 into the housing space 128 of the housing body 120a is formed on the base end side of the housing body 120a in the longitudinal direction. The base end opening 125 of the housing body 120a is closed by a lid member (not shown) with the chassis 127 housed in the housing space 128.

A bottom surface 121 of the housing body 120a is provided with a sheet-shaped adhesive part (not shown) that can adhere to the body surface H of the user. In an initial state before the drug solution administration device 100 is attached to the user, a releasable protective sheet is attached to an adhesive surface of the adhesive part.

As shown in Fig. 4, the chassis 127 holds the drug solution container 110, the plunger 130, a drive mechanism 140 for driving the forward movement of the plunger 130, a control unit 160, and a power supply unit 170.

A main body 131 of the plunger 130 is inserted into an inner cavity 111a of the main body 111 of the drug solution container 110 (see Fig. 4). A gasket 135 is provided at the tip of the main body 131 of the plunger 130. The gasket 135 can be made of, for example, a resin material such as a rubber material or an elastomer. The outer periphery of the gasket 135 is in liquid-tight contact with the inner peripheral surface of the main body 111 of the drug solution container 110, whereby the base end side of the gasket 135 is liquid-tightly sealed.

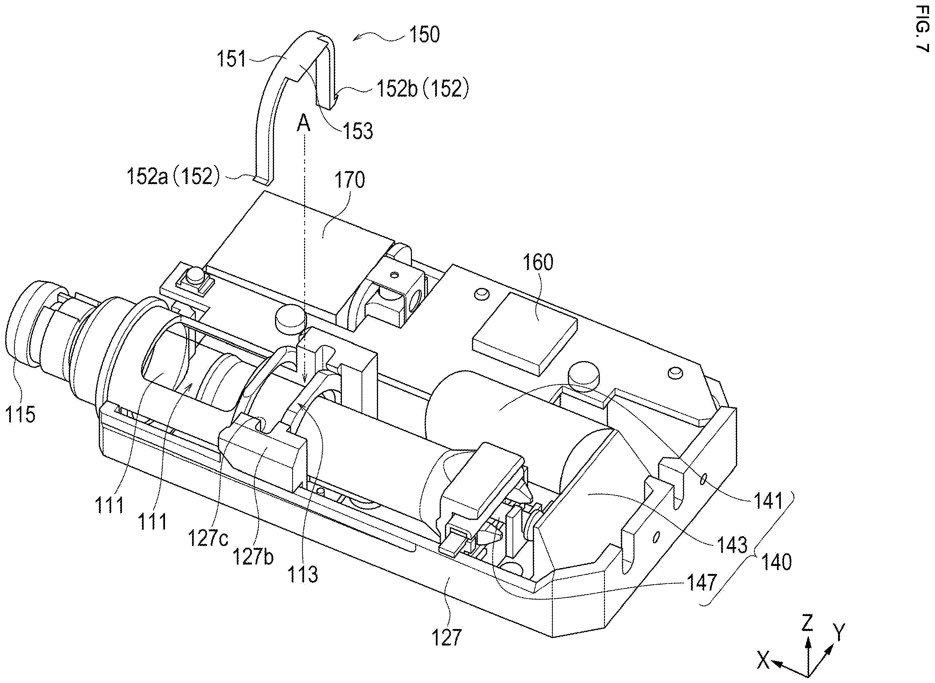

A flange 113 that is wider than the main body 111 of the drug solution container 110 in the radial direction is formed at the base end of the drug solution container 110 (see Fig. 7).

The drug solution container 110 is a so-called prefilled type drug solution container. Therefore, the drug solution is filled in advance in the inner cavity 111a of the main body 111 of the drug solution container 110. Examples of the drug solution include protein preparations, narcotic analgesics, and diuretics.

A sealing member (not shown) for preventing the drug solution from leaking is disposed at a tip opening (discharge port) of the drug solution container 110. As shown in Fig. 3, the drug solution container 110 is disposed such that the tip opening projects from the housing body 120a to the outside. In addition, a mounting part 115 that is to be connected to a tube 240 (see Fig. 1) described later is attached to a portion of the drug solution container 110 that protrudes from the housing body 120a.

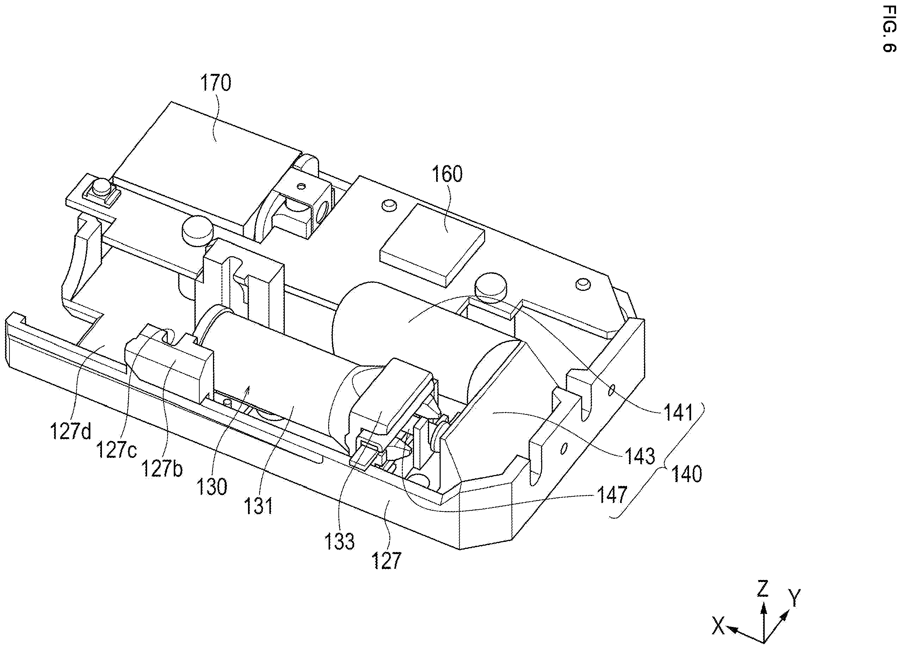

As shown in Fig. 6, the chassis 127 includes a support surface 127d that supports the bottom surface of the main body 111 of the drug solution container 110 (the outer peripheral surface of a certain range located on the chassis 127 side in the circumferential direction). Further, as shown in Fig. 7, the chassis 127 has a flange support part 127b that supports the flange 113 of the drug solution container 110. The flange support part 127b includes a groove 127c into which the flange 113 of the drug solution container 110 can be inserted.

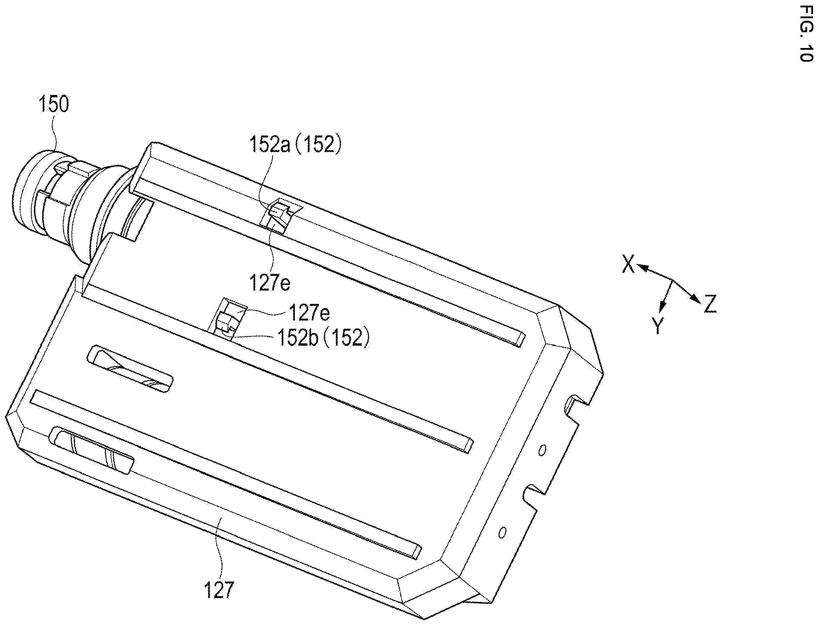

As shown in Fig. 10, the back surface of the chassis 127 (the surface reverse to the surface having the support surface 127d on which the drug solution container 110 and the like are placed) has holes 127e into which tabs 152a and 152b of the fixing member 150 described later can be fitted.

The control unit 160 controls the operation of the drug solution administration device 100 for delivering the drug solution. The control unit 160 can be achieved by, for example, a known microcomputer (electronic circuit element) mounted with a CPU, a RAM, a ROM and the like.

The power supply unit 170 can be composed of, for example, a known battery.

As shown in Fig. 4, the drive mechanism 140 includes a motor 141 that receives a drive current from the power supply unit 170 to generate a rotational drive force, a reduction mechanism 143 that includes a gear that transmits the rotational drive force of the motor 141, and the like, and a feed screw 147 connected to the reduction mechanism 143.

The feed screw 147 is connected to a base end connection part 133 disposed near the base end of the plunger 130. The feed screw 147 converts the rotary motion transmitted from the reduction mechanism 143 into a linear motion, and advances the plunger 130 in the longitudinal direction (X direction). The plunger 130 expels the drug solution from the inner cavity 111a of the main body 111 of the drug solution container 110 to the tube 240 (see Fig. 1) due to the movement toward the tip of the drug solution container 110.

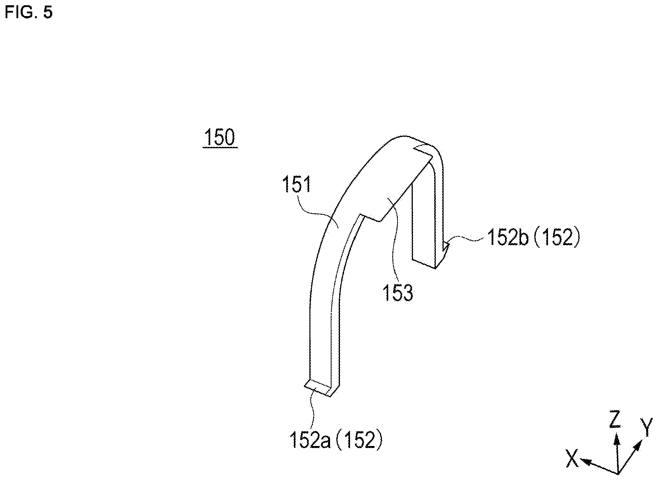

Next, the fixing member 150 will be described with reference to Fig. 5.

As shown in Fig. 5, the fixing member 150 includes a guide part 151 and an engagement part 152. The guide part 151 is mounted along the main body 111 of the drug solution container 110 from the side reverse to the bottom surface of the main body 111 of the drug solution container 110 in the circumferential direction (from an upper surface side reverse to the surface of the drug solution container 110 facing the chassis 127). The engagement part 152 is engageable with the chassis 127.

The guide part 151 of the fixing member 150 has a curved shape so as to be attached along the outer peripheral surface of the main body 111 of the drug solution container 110. Further, the guide part 151 has a protrusion 153 protruding in a predetermined direction.

The engagement part 152 of the fixing member 150 has a first tab 152a and a second tab 152b. As shown in Fig. 10, the first tab 152a and the second tab 152b are configured to be engageable with the holes 127e formed in the chassis 127.

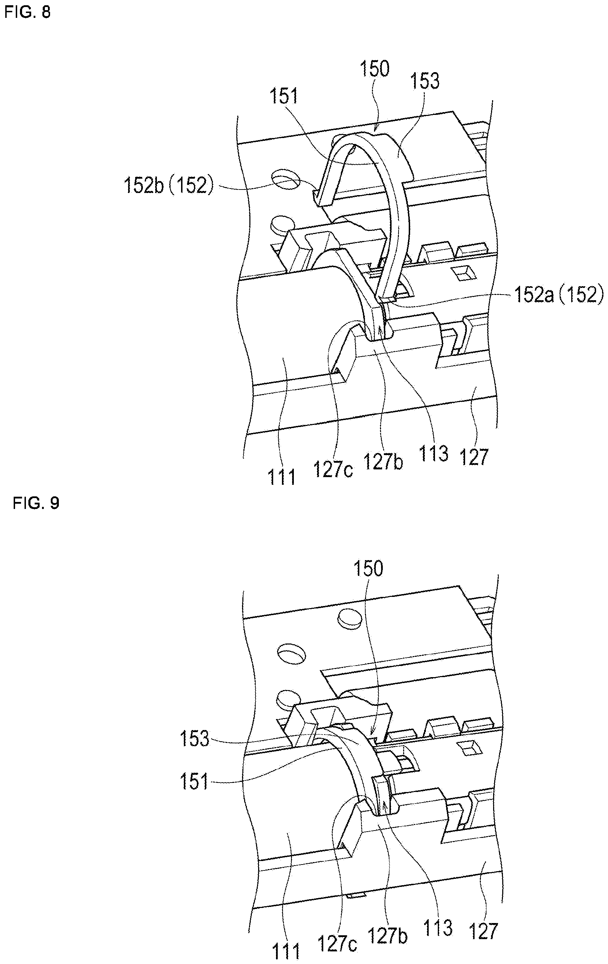

As shown in Fig. 9, while the flange 113 of the drug solution container 110 is inserted in the groove 127c of the flange support part 127b of the chassis 127, the guide part 151 of the fixing member 150 is mounted on the tip side of the flange 113 so as to hold the flange 113 with the flange support part 127b (with the surrounding inner wall of the groove 127c).

Next, a procedure for mounting the fixing member 150 will be described.

First, as shown in Fig. 6, the components other than the drug solution container 110 is attached to the chassis 127.

Next, as shown in Fig. 7, the drug solution container 110 is attached to the chassis 127. At this time, the bottom surface of the main body 111 of the drug solution container 110 is placed on the support surface 127d of the chassis 127. Then, as shown in Fig. 7, the fixing member 150 is mounted to the chassis 127. As indicated by an arrow A in Fig. 7, the fixing member 150 is mounted to the chassis 127 from the side opposite to the side where the bottom surface of the drug solution container 110 is placed.

When the fixing member 150 is mounted, the guide part 151 of the fixing member 150 is mounted along the outer surface of the main body 111 of the drug solution container 110 as shown in Figs. 8 and 9. Further, the guide part 151 is mounted on the tip side of the flange 113. The protrusion 153 of the fixing member 150 is mounted so as to cover the flange 113 of the drug solution container 110 from the upper surface side.

As shown in Fig. 10, the fixing member 150 is fixed to the chassis 127 by engagement between the tabs 152a and 152b and the holes 127e formed in the chassis 127. The drug solution container 110 is fixed to the chassis 127 by means of the fixing member 150.

While the drug solution administration device 100 is delivering the drug solution, the bottom surface of the main body 111 of the drug solution container 110 is supported by the chassis 127, and the upper surface of the main body 111 of the drug solution container 110 is fixed by the fixing member 150. Therefore, even when the plunger 130 and the drug solution container 110 are excessively pressed because of thrust generated due to the forward movement of the plunger 130 within the drug solution container 110, the drug solution administration device 100 can press the plunger 130 and the drug solution container 110 in a direction intersecting the longitudinal direction of the drug solution container 110 (direction orthogonal to the direction of the forward movement of the plunger 130). Therefore, the drug solution administration device 100 can adequately prevent the drug solution container 110 and the plunger 130 from bending in an inverted V shape.

### (Administration instrument)

As shown in Figs. 1 and 2, the administration instrument 200 is connectable to the drug solution administration device 100.

The administration instrument 200 includes a connector 210, a needle tube 220 that is inserted into a living body, a puncture part (cannula housing) 230, a tube 240, and a puncture assistance tool 250 that assists the puncture of the living body with the needle tube 220.

The connector 210 is connectable to the drug solution administration device 100 via a mounting part 215 fixed to the connector 210. The mounting part 215 can be connected to the drug solution administration device 100 by being externally fitted to the mounting part 115 (see Fig. 6) which is provided near the tip of the drug solution container 110 and protrudes to the outside of the housing 120.

The mounting part 215 has inside a connecting needle (not shown) capable of piercing a sealing member (not shown) provided at the tip of the drug solution container 110. The tube 240 communicates with the inner cavity 111a of the main body 111 of the drug solution container 110 via the connecting needle.

A flow path (not shown) that connects the tube 240 and the inner cavity of the needle tube 220 is formed inside the puncture part 230. The drug solution delivered to the puncture part 230 via the tube 240 is administered into the living body through the flow path formed inside the puncture part 230 and the needle tube 220.

When the drug solution is delivered to the user, the puncture assistance tool 250 is attached to the puncture part 230. The puncture assistance tool 250 holds an introducer needle (inner needle) 251. The introducer needle 251 projects from the tip of the needle tube 220 when the puncture assistance tool 250 is attached to the puncture part 230. The user can insert the needle tube 220 into the living body, while preventing the needle tube 220 from having any troubles such as breakage, by piercing the living body with the needle tube 220 with the introducer needle 251 inserted into the needle tube 220.

The puncture assistance tool 250 is removed from the puncture part 230 after the needle tube 220 is inserted into the living body. The introducer needle 251 is withdrawn from the inner cavity of the needle tube 220 when the puncture assistance tool 250 is removed from the puncture part 230.

After the needle tube 220 is inserted into the living body, the puncture assistance tool 250 is removed, and the puncture part 230 is left on the body surface H of the user with the needle tube 220 left in the living body. In this state, the plunger 130 of the drug solution administration device 100 advances in the drug solution container 110, so that the drug solution filled in the drug solution container 110 is delivered to the inner cavity of the needle tube 220 through the tube 240 and the flow path of the puncture part 230.

The introducer needle 251 can be, for example, a metal needle. Further, the needle tube 220 can be composed of, for example, a tubular member (cannula) made of resin.

Similar to the drug solution administration device 100, the administration instrument 200 is of a patch type that is attached to the body surface H of the user when used. The contact surface (bottom surface) 231 of the puncture part 230 of the administration instrument 200 is provided with a sheet-shaped adhesive part (not shown) that can adhere to the body surface. In an initial state before the administration instrument 200 is attached to the user, a releasable protective sheet is attached to an adhesive surface of the adhesive part.

As described above, the drug solution administration device 100 according to the present embodiment includes the drug solution container 110 having the cylindrical main body 111 filled with a drug solution, the housing 120 for holding the drug solution container 110, the plunger 130 for expelling the drug solution in the drug solution container 110, and the fixing member 150 for fixing the drug solution container 110 to the housing 120. The housing 120 includes the chassis 127 having the support surface 127d that supports the bottom surface of the main body 111 of the drug solution container 110. The fixing member 150 includes the guide part 151 that is mounted along the main body 111 of the drug solution container 110 from the side reverse to the bottom surface of the main body 111 of the drug solution container 110 in the circumferential direction, and the engagement part 152 that is engageable with the chassis 127 of the housing 120.

The drug solution administration device 100 configured as described above delivers the drug solution, while the bottom surface of the main body 111 of the drug solution container 110 is supported by the chassis 127, and the upper surface of the main body 111 of the drug solution container 110 is fixed by the fixing member 150.
Therefore, it is possible to prevent deformation resulting in bending of the plunger 130 and the drug solution container 110 during delivery of the drug solution.

Further, in the drug solution administration device 100, the guide part 151 of the fixing member 150 has a curved shape so as to be attached along the outer peripheral surface of the main body 111 of the drug solution container 110. Therefore, the fixing member 150 can rigidly fix the outer peripheral surface of the main body 111 of the drug solution container 110 by the guide part 151.

Further, in the drug solution administration device 100, the chassis 127 included in the housing 120 has the holes 127e, and the engagement part 152 of the fixing member 150 has the tabs 152a and 152b that can be fitted into the holes 127e. Therefore, the fixing member 150 can be easily and reliably fixed to the chassis 127 by means of the tabs 152a and 152b.

Further, in the drug solution administration device 100, the flange 113 that is wider than the main body 111 of the drug solution container 110 in the radial direction is formed at the base end of the drug solution container 110. The chassis 127 included in the housing 120 has the flange support part 127b that supports the flange 113 of the drug solution container 110. Further, the guide part 151 of the fixing member 150 is mounted on the tip side of the flange 113 so as to hold the flange 113 of the drug solution container 110 with the flange support part 127b. Due to the flange 113 of the drug solution container 110 being held between the fixing member 150 and the flange support part 127b as described above, the fixing member 150 can more reliably fix the drug solution container 110 to the chassis 127.

While the drug solution administration device according to the present invention has been described above by way of the embodiment, the present invention is not limited to each of the described configurations, and can be appropriately modified on the basis of the description of the claims. i.e. the scope of the invention is defined by the appended claims.

Further, for example, the engagement part can be configured to be engageable with the housing by a structure other than the structure using the tabs described in the embodiment.

### Reference Signs List

10 Drug solution administration system
100 Drug solution administration device
110 Drug solution container
111 Main body of drug solution container
111a Inner cavity of main body of drug solution container
113 Flange of drug solution container
120 Housing
120a Housing body
121 Bottom surface of housing body
123 Upper surface of housing body
125 Base end opening of housing body
127 Chassis (support part)
127b Flange support part
127c Groove
127d Support surface
127e Hole
128 Housing space
130 Plunger
131 Main body of plunger
140 Drive mechanism
150 Fixing member
151 Guide part
152 Engagement part
152a First tab (tab)
152b Second tab (tab)
153 Protrusion
200 Administration instrument
H Body surface

## Claims

1. A drug solution administration device (100) comprising:
a drug solution container (110) that has a main body (111) having a cylindrical shape and filled with a drug solution;
a housing (120) that holds the drug solution container (110);
a plunger (130) that expels the drug solution in the drug solution container (110); and
a fixing member (150) that fixes the drug solution container (110) to the housing (120),
wherein the housing (120) includes a support part (127) having a support surface that supports a bottom surface of the main body (111) of the drug solution container (110), and
the fixing member (150) includes a guide part (151) mounted along the main body (111) of the drug solution container (110) from a side opposite to the bottom surface of the main body (111) of the drug solution container (110) in a circumferential direction of the cylindrical main body, and an engagement part (152) engageable with the support part (127) of the housing (120),
the drug solution container (110) has, at a base end, a flange (113) wider than the main body (111) of the drug solution container (110) in a radial direction,
**characterized in that**
the housing (120) has a flange support part (127b) that supports the flange (113), and
the guide part (151) of the fixing member (150) is disposed on a tip side of the flange (113) so as to hold the flange (113) with the flange support part (127b).

2. The drug solution administration device (100) according to claim 1, wherein the guide part (151) has a curved shape so as to be mounted along an outer peripheral surface of the main body (111) of the drug solution container (110).

3. The drug solution administration device (100) according to claim 1 or 2,
wherein the housing (120) has a hole formed in the support part (127), and
the engagement part (152) of the fixing member (150) has a tab engageable with the hole.

4. The drug solution administration device (100) according to any one of claims 1 to 3,
wherein the guide part (151) has a protrusion (153) protruding in a predetermined direction.

## Patentansprüche

1. Vorrichtung zur Verabreichung einer Arzneimittellösung (100), mit:
einem Arzneimittellösungsbehälter (110), der einen Hauptkörper (111) mit einer zylindrischen Form aufweist und mit einer Arzneimittellösung gefüllt ist;
einem Gehäuse (120), das den Arzneimittellösungsbehälter (110) aufnimmt;
einem Kolben (130), der die Arzneimittellösung in dem Arzneimittellösungsbehälter (110) ausstößt; und
einem Befestigungselement (150), das den Arzneimittellösungsbehälter (110) an dem Gehäuse (120) fixiert,
wobei das Gehäuse (120) ein Trägerteil (127) mit einer Trägerfläche aufweist, die eine Bodenfläche des Hauptkörpers (111) des Arzneimittellösungsbehälters (110) hält, und
das Befestigungselement (150) ein Führungsteil (151) hat, das entlang des Hauptkörpers (111) des Arzneimittellösungsbehälters (110) von einer Seite gegenüber der Bodenfläche des Hauptkörpers (111) des Arzneimittellösungsbehälters (110) in einer Umfangsrichtung des zylindrischen Hauptkörpers angebracht ist, und ein Eingriffsteil (152), das mit dem Trägerteil (127) des Gehäuses (120) in Eingriff gebracht werden kann,
der Arzneimittellösungsbehälter (110) an einem Bodenende einen Flansch (113) aufweist, der in einer radialen Richtung breiter als der Hauptkörper (111) des Arzneimittellösungsbehälters (110) ist,
**dadurch gekennzeichnet, dass**
das Gehäuse (120) ein Flanschstützteil (127b) aufweist, das den Flansch (113) hält, und
das Führungsteil (151) des Befestigungselements (150) an einer Stirnseite des Flansches (113) vorgesehen ist, um den Flansch (113) mit dem Flanschstützteil (127b) zu halten.

2. Vorrichtung (100) zur Verabreichung von Arzneimittellösungen gemäß Anspruch 1, wobei das Führungsteil (151) eine gekrümmte Form aufweist, um entlang einer außenliegenden Umfangsfläche des Hauptkörpers (111) des Arzneimittellösungsbehälters (110) angebracht zu sein.

3. Vorrichtung (100) zur Verabreichung einer Arzneimittellösung gemäß Anspruch 1 oder 2,
wobei das Gehäuse (120) ein im Stützteil (127) ausgebildetes Loch aufweist, und
das Eingriffsteil (152) des Befestigungselements (150) eine mit dem Loch in Eingriff bringbare Lasche aufweist.

4. Vorrichtung (100) zur Verabreichung von Arzneimittelösungen gemäß einem der Ansprüche 1 bis 3,
wobei das Führungsteil (151) einen Vorsprung (153) aufweist, der in eine vorbestimmte Richtung vorsteht.

## Revendications

1. Dispositif d'administration de solution médicamenteuse (100) comprenant :
un contenant de solution médicamenteuse (110) qui possède un corps principal (111) de forme cylindrique et rempli d'une solution médicamenteuse ;
un boîtier (120) qui contient le contenant de solution médicamenteuse (110) ;
un piston (130) qui expulse la solution médicamenteuse dans le contenant de solution médicamenteuse (110) ; et
un élément de fixation (150) qui fixe le contenant de solution médicamenteuse (110) au boîtier (120),
dans lequel le boîtier (120) comporte une partie de support (127) ayant une surface de support qui supporte une surface inférieure du corps principal (111) du contenant de solution médicamenteuse (110), et
l'élément de fixation (150) comporte une partie de guidage (151) montée le long du corps principal (111) du récipient de solution médicamenteuse (110) à partir d'un côté opposé à la surface inférieure du corps principal (111) du récipient de solution médicamenteuse (110) dans une direction circonférentielle du corps principal cylindrique, et une partie d'engagement (152) pouvant être engagée avec la partie de support (127) du boîtier (120),
le récipient de solution médicamenteuse (110) possède, à une extrémité de base, une bride (113) plus large que le corps principal (111) du récipient de solution médicamenteuse (110) dans une direction radiale, **caractérisé en ce que**
le boîtier (120) a une partie de support de bride (127b) qui supporte la bride (113), et
la partie de guidage (151) de l'élément de fixation (150) est disposée sur un côté de pointe de la bride (113) de manière à maintenir la bride (113) avec la partie de support de bride (127b).

2. Dispositif d'administration de solution médicamenteuse (100) selon la revendication 1, dans lequel la partie de guidage (151) a une forme incurvée de manière à être montée le long d'une surface périphérique extérieure du corps principal (111) du récipient de solution médicamenteuse (110).

3. Dispositif d'administration de solution médicamenteuse (100) selon la revendication 1 ou 2,
dans lequel le boîtier (120) a un trou formé dans la partie de support (127), et
la partie d'engagement (152) de l'élément de fixation (150) comporte une languette pouvant s'engager dans le trou.

4. Dispositif d'administration de solution médicamenteuse (100) selon l'une des revendications 1 à 3,
dans lequel la partie de guidage (151) présente une protubérance (153) faisant saillie dans une direction prédéterminée.
